Europäisches Patentamt

European Patent Office

Office européen des brevets

(11) Numéro de publication: **0 252 855 B1**

# FASCICULE DE BREVET EUROPEEN

(45) Date de publication de fascicule du brevet: **31.03.93**  (51) Int. Cl.⁵: **C12P  17/18**

(21) Numéro de dépôt: **87401650.4**

(22) Date de dépôt: **10.07.87**

(54) **Procédé de bioconversion sélective du dinitrate d'isosorbitol.**

(30) Priorité: **11.07.86 FR 8610177**

(43) Date de publication de la demande:
**13.01.88 Bulletin  88/02**

(45) Mention de la délivrance du brevet:
**31.03.93 Bulletin  93/13**

(84) Etats contractants désignés:
**AT BE CH DE ES FR GB GR IT LI LU NL SE**

(56) Documents cités:

**CHEMICAL ABSTRACTS, vol. 107, no. 9, 31 août 1987, page 557, résumé no. 76009h, Columbus, Ohio, US; J.S. Ropenga et al.: "Bioconversion of isosorbide dinitrate into isosorbide mononitrade by the protozoan Tetrahymena thermophila: relationship to glutathione transferase levels", & APPL. MICROBIOL. BIOTECHNOL. 1987, 26(2), 117-19**

**JOURNAL OF PHARMACEUTICAL SCIENCES, vol. 63, no. 7, juillet 1974, pages 1147-1149; W.H. DOWN et al.: "Biotransformation of isosorbide dinitrate in humans"**

(73) Titulaire: **CENTRE NATIONAL DE LA RECHERCHE SCIENTIFIOUE (CNRS)**
**15, Ouai Anatole France**
**F-75700 Paris Cedex 07(FR)**

Titulaire: **SOCIETE NATIONALE DES POUDRES ET EXPLOSIFS**
**12, quai Henri IV**
**F-75181 Paris Cédex 04(FR)**

(72) Inventeur: **Lenfant, Maryse**
**18, rue des Chataigniers**
**F-91190 Gif Sur Yvette(FR)**
Inventeur: **Ropenga, Jacek**
**4, allée des Bathes, Apt. 60**
**F-91940 Les Ulis(FR)**
Inventeur: **Wimmer, Eric**
**2B Ch. des Chênes Verts**
**F-84700 Sorgues(FR)**

(74) Mandataire: **Warcoin, Jacques et al**
**Cabinet Régimbeau 26, avenue Kléber**
**F-75116 Paris (FR)**

CHEMICAL ABSTRACTS, vol. 101, no. 3, 16 juillet 1984, page 17, résumé no. 16774v, Columbus, Ohio, US; S.Y.P. KING et al.: "Rapid microbial degradation of organic nitrates in rat excreta. Reexamination of the urinary and fecal metabolite profiles of pentaerythritol tetranitrate in the rat", & DRUG METAB. DISPOS. 1984, 12(3), 353-7

**Description**

La présente invention concerne la bioconversion sélective des fonctions ester nitrique en fonctions hydroxy dü dinitrate d'isosorbitol (DINIS)

On distingue deux mononitrates d'isosorbitol qui sont dénommés MONIS. Lorsque la fonction hydroxy est en position exo, le composé est dénommé MONIS-5, et, lorsque cette fonction est en position endo, MONIS-2.

Les MONIS sont connus comme vasodilatateurs coronaires à l'instar du dinitrate d'isosorbitol (DINIS) et de la nitroglycérine.

Les MONIS sont actuellement préparés par des procédés qui présentent des inconvénients. La synthèse par nitration de l'isosorbitol nécessite l'utilisation d'un mélange de nitration $HNO_3$ - anhydride acétique - acide acétique pouvant conduire à la formation de nitrate d'acétyle considéré comme un explosif plus sensible que la nitroglycérine.

Un autre procédé consiste à préparer les acétates d'isosorbitol puis à les nitrer ; là encore, les risques d'explosion sont importants et le procédé long et de rendement faible.

De nouveaux procédés ont permis de limiter les risques dans la préparation de ce type de composés mais sans pour autant être parfaitement satisfaisants sur le plan de la sécurité et/ou du rendement, comme cela ressort du brevet français 2 500 835.

C'est pourquoi des techniques douces de préparation de ces composés mettant en oeuvre des bioconversions présentent un grand intérêt.

Le procédé selon la présente invention est plus particulièrement destiné à la bioconversion sélective de DINIS en MONIS-5, et est caractérisé en ce que le DINIS est ajouté à un milieu contenant au moins un microorganisme possédant une gluthation transférase ou un extrait acellulaire d'un tel microorganisme, de préférence d'ailleurs le milieu en cause sera un milieu de culture du microorganisme.

Parmi les microorganismes utilisables dans le cadre de la présente invention, il faut citer plus particulièrement les protozoaires, les fongis et les bactéries. Parmi les protozoaires, il faut citer plus particulièrement Tetrahymena, et, parmi les fongis, il faut citer plus particulièrement Beauveria, Aspergillus, Streptomyces et Cunninghamella.

La fermentation de ces microorganismes dans un milieu contenant du DINIS conduit à la formation de MONIS, avec une prépondérance du MONIS-5 par rapport au MONIS-2.

Bien que la bioconversion du DINIS puisse être effectuée dans un milieu ne contenant que les cellules des microorganismes, il est préférable d'effectuer cette bioconversion dans un milieu de culture. Ce milieu de culture sera, de préférence, un milieu minimum contenant, de préférence, 5 à 20 g/l de glucose, des extraits de levure, et la bioconversion sera effectuée à une température de l'ordre de 20 à 30°C à un pH voisin de la neutralité.

Les expériences mises en oeuvre ont permis de montrer que l'addition d'acides aminés dans le milieu de culture augmentait nettement le rendement en MONIS-5.

Dans les conditions précédentes, une concentration en DINIS comprise entre 1 et 5 g/l est utilisable.

Il est, en outre, préférable d'utiliser, lorsque cela est possible pour les fongis, un mycelium déjà âgé qui donne des rendements de bioconversion supérieurs. Bien entendu, lorsque l'on utilise un milieu de croissance, il est nécessaire de prévoir une aération du milieu.

Comme cela a été indiqué précédemment, il est également possible de prévoir d'utiliser le mycelium dans un milieu qui ne soit pas un milieu de croissance, on peut même utiliser, dans certains cas, des extraits partiellement débarrassés de structure cellulaire, ceci facilite les opérations ultérieures de séparation.

Dans certains cas, les microorganismes pourront être utilisés sous forme immobilisée, par exemple par adsorption sur un support ou par encapsulation dans un polymère, agar par exemple.

Les conditions de mise en oeuvre de cette bioconversion permettent d'écarter tout risque lié à la manipulation de ce type de produit et permettent d'obtenir des rendements en MONIS-5 très importants, comme cela ressortira des exemples ci-après.

Le DINIS utilisé dans le cadre de la présente invention à titre de produit de départ est un produit explosible, mais il est possible de le manipuler en toute sécurité dans la mesure où ce produit est humide ou imprégné de solvant ou en solution.

Les exemples ci-après sont destinés à mettre en évidence d'autres avantages et caractéristiques de la présente invention.

## EXEMPLE 1

Bioconversion du DINIS par des cultures de Cunninghemella echinulata (C. ech.) NRRL 3655 et Cunninghamella elegans (C. el.) ATCC 3245

Les pré-cultures sont effectuées en tube d'agar incliné, à 24° sur gélose de pomme de terre (I.P.P.). Les cultures (50 ml) sont réalisées en milieu liquide "farine de soja" dans des erlenmeyers de 250 ml, agités (180 tours/min.) à 24°C. Les ensemencements sont effectués par addition de 0,5 à 1 ml d'une suspension de spores à la densité moyenne de $3.10^5$ spores/ml. Après 44 heures de croissance, et selon les essais, le DINIS est ajouté soit à la culture, soit à la suspension de mycelium dans du tampon phosphate 0,05 M pH 7, et incubé à 24°C pendant les périodes de temps choisies.

Différentes conditions de biotransformation ont été étudiées :

a) Incubation en milieu de culture, le DINIS est ajouté à 1 g/l en solution dans le méthanol (50 mg/ml).

b) Incubation en tampon phosphate 0,05 M pH 7, le DINIS est ajouté à 1 g/l en solution dans le méthanol (50 mg/ml).

c) Incubation en tampon phosphate 0,05 M pH 7, le DINIS est ajouté en poudre à 1 g/l.

Les quantités de MONIS-5 et MONIS-2 formées au cours du temps, par bioconversion, ont été estimées par analyse par HPLC sur colonne de $\mu$Bondapack C-18.

Les résultats obtenus sont rassemblés pour C. ech. au tableau 1 et pour C. el. au tableau 2.

<u>**TABLEAU 1**</u>

<u>**Biotransformation du DINIS par Cunninghamella echinulata**</u>
<u>**NRRL 3655 (ATCC 36190)**</u>

a) Incubation en milieu de culture ; DINIS 1 g/l dans le méthanol (50 mg/ml) ; âge des cultures 44 heures ; quantité de mycelium : 73 g/l ;

| Heures | | 4 | 8 | 21 | 28 | 33 | 46 | 55 | 73 | 100 |
|---|---|---|---|---|---|---|---|---|---|---|
| DINIS | (mg/l) | 595 | 570 | 535 | 473 | 445 | 351 | 339 | 262 | 182 |
| MONIS-2 | (mg/l) | 8 | 26 | 56 | 66 | 73 | 83 | 100 | 104 | 103 |
| MONIS-5 | (mg/l) | 27 | 64 | 134 | 175 | 186 | 232 | 239 | 268 | 197 |

b) Incubation en tampon phosphate 0,05 M, pH 7 ; DINIS 1 g/l dans le méthanol (50 mg/l) ; âge des cultures 44 heures ; quantité de mycelium : 80 g/l ;

| Heures | | 4 | 8 | 21 | 33 | 46 | 73 | 120 |
|---|---|---|---|---|---|---|---|---|
| DINIS | (mg/l) | 507 | 655 | 442 | 443 | 394 | 328 | 272 |
| MONIS-2 | (mg/l) | 10 | 24 | 27 | 62 | 76 | 83 | 103 |
| MONIS-5 | (mg/l) | 26 | 51 | 108 | 148 | 198 | 209 | 227 |

c) Incubation en tampon phosphate 0,05 M, pH 7 ; DINIS 1 g/l en poudre ; âge des cultures 44 heures ; quantité de mycelium 101 g/l ;

| Heures | | 8 | 21 | 33 | 46 | 73 |
|---|---|---|---|---|---|---|
| DINIS | (mg/l) | 605 | 533 | 510 | 480 | 406 |
| MONIS-2 | (mg/l) | 15 | 42 | 48 | 56 | 82 |
| MONIS-5 | (mg/l) | 30 | 64 | 101 | 136 | 145 |

Concentration initiale de DINIS : Les concentrations évaluées en DINIS, MONIS-2 et MONIS-5 n'ont pas été corrigées. (Une évaporation de l'ordre de 10 à 15 % doit être prise en compte).

## TABLEAU 2

### Biotransformation du DINIS par Cunninghamella elegans (ATCC 9245)

a) Incubation en milieu de culture ; DINIS 1 g/l dans le méthanol (50 mg/ml) ; âge des cultures: 44 heures ; quantité de mycelium : 73 g/l ;

| Heures | | 4 | 21 | 33 | 46 | 55 | 73 | 120 |
|---|---|---|---|---|---|---|---|---|
| DINIS | (mg/l) | 678 | 545 | 441 | 242 | 265 | 123 | 14,5 |
| MONIS-2 | (mg/l) | 18 | 112 | 165 | 185 | 314 | 305 | 170 |
| MONIS-5 | (mg/l) | 26 | 99 | 121 | 160 | 214 | 230 | 188 |

b) Incubation en tampon phosphate 0,05 M, pH 7 ; DINIS 1 g/l dans le méthanol (50 mg/ml) ; âge des cultures: 44 heures ; quantité de mycelium : 80 g/l ;

| Heures | | 4 | 8 | 21 | 33 | 46 | 73 |
|---|---|---|---|---|---|---|---|
| DINIS | (mg/l) | 631 | 625 | 517 | 463 | 344 | 325 |
| MONIS-2 | (mg/l) | 13 | 19 | 63 | 98 | 115 | 149 |
| MONIS-5 | (mg/l) | 23 | 32 | 71 | 102 | 115 | 126 |

Concentration initiale de DINIS : Les concentrations évaluées en DINIS, MONIS-2 et MONIS-5 n'ont pas été corrigées. (Une évaporation de l'ordre de 10 à 15 % doit être prise en compte).

1. Les deux souches C. ech. et C. el. convertissent le DINIS en MONIS-5 et MONIS-2.
2. Le meilleur taux de conversion est observé lorsque la souche C. ech. est incubée en milieu de culture pendant 73 heures. Un temps d'incubation prolongé conduit à la chute du rendement global (DINIS + MONIS-5 + MONIS-2) vraisemblablement par conversion ultérieure des dérivés mononitrés en isosorbide.

Dans ces conditions, on obtient avec un rendement global de 58 % le mélange DINIS, MONIS-5, MONIS-2. Les dérivés mononitrés sont formés avec des rendements respectifs de 33 % en MONIS-5 et 13 % environ en MONIS-2 (rendement calculé sur la quantité de DINIS consommée), soit un rapport :

$$\frac{MONIS-5}{MONIS-2} = 2,6$$

Afin d'améliorer les rendements de conversion,des essais ont été effectués sur une quantité de DINIS réduite à 500 mg/l ; les résultats obtenus sont rassemblés dans le tableau 3 ci-après :

TABLEAU 3

| Biotransformation du DINIS par Cunninghamella echinulata NRRL 3655 (ATCC 36190) | | | | | | |
|---|---|---|---|---|---|---|
| Heures | Essai | 48 | 69 | 93 | 116 | 140 |
| DINIS (mg/l)* | 1 | 268 | 169 | 107 | 68 | 60 |
| | 2 | 270 | 169 | 116 | 86 | 75 |
| MONIS-5 (mg/l)* | 1 | 143 | 190 | 216 | 218 | 244 |
| | 2 | 150 | 197 | 219 | 225 | 238 |
| MONIS-2 (mg/l)* | 1 | 61 | 67 | 95 | 71 | 93 |
| | 2 | 58 | 89 | 98 | 106 | 96 |
| DINIS 500 mg/l dans le méthanol (50 mg/ml) ; incubation en milieu de culture ; âge des cultures : 44 heures ; quantité moyenne de mycelium : 78 g/l ; | | | | | | |

\* Les concentrations évaluées en DINIS, MONIS-5 et MONIS-2 n'ont pas été corrigées.
(L'évaporation de l'ordre de 10 à 15 % doit être prise en compte).

Dans ces conditions, il est possible d'obtenir, pour des périodes d'incubation variant de 63 à 93 heures, le mélange (DINIS + MONIS-5 + MONIS-2) avec un rendement global de 80-90 %. Les dérivés mononitrés MONIS-5 et MONIS-2 sont formés avec des rendements respectifs de 50 et 22 % (calculés sur la quantité de DINIS consommée).

Contrairement à la souche C. ech. qui conduit à la formation préférentielle de MONIS-5, la souche C. el. effectue la conversion du DINIS préférentiellement en isomère 2.

Ces résultats indiquent que dans une même famille de Phycomycetes, les différentes souches sont susceptibles de contenir, en proportions variables, des enzymes de la famille des glutathions transférase qui spécifiquement doivent permettre la conversion du DINIS en chacun des isomères MONIS-5 et MONIS-2.

EXEMPLE 2

Essais de mutagénèse de Cunninghamella echinulata Mise au point d'une mutagénèse à l'aziridine (éthylèneimine)

Après différentes tentatives, il est apparu que le traitement de spores de C. ech. pendant 4 minutes avec une solution léthale à 5 % d'aziridine induisait la formation de mutants détectables après 72 heures d'incubation sur gélose.

Conditions de mutagénèse

Les spores de C. ech. prélevées sur 2 tubes inclinés de pré-cultures ont été lavées avec 8 ml d'eau (4 ml/tube) et remises en suspension dans 4,75 ml d'eau auxquels sont additionnés 0,25 ml d'aziridine (solution finale 5 %).

Après 4 minutes des échantillons (0,5 ml) ont été prélevés, dilués ($10^2$, $10^3$, $10^4$ fois) puis ensemencés sur des boîtes de Petri de gélose de pomme de terre à raison de 0,2 ml/boîte.

Les résultats obtenus montrent que :

1. La concentration en spores de la solution avant traitement est de $2 \times 10^5$ spores/ml et de $3 \times 10^3$ spores/ml (1,5 %) après traitement.

2. La possibilité d'isoler après 72 heures de cultures, des souches mutées.

L'efficacité des mutants isolés pour effectuer la biotransformation du DINIS a été évaluée en milieu liquide dans les conditions classiques.

Résultats

15 mutants ont été isolés et cultivés sur agar incliné. Les mutants ont été évalués pour leur capacité à biotransformer le DINIS, dans les conditions choisies pour C. ech. (mycelium de 44 heures). La biotransformation a été évaluée après 48 heures d'incubation.

Il apparaît :

1. Qu'il est possible de faire varier dans les souches, par mutagénèse, le taux de bioconversion de MONIS-2 et MONIS-5 tel que le rapport MONOS-5/MONIS-2 varie de 2,07 à 2,91, donc le taux des enzymes responsables de la bioconversion.

2. Que 2 mutants sont plus efficaces que la souche de départ pour obtenir préférentiellement le MONIS-5 à partir du DINIS, un mutart conduisant préférentiellement à l'isomère 2.

3. Que deux mutants effectuent la bioconversion avec un rendement global (DINIS + MONIS-5 + MONIS-2) quantitatif.

EXEMPLE 3

Bioconversion du DINIS par diverses souches de Beauveria, Aspergillus et Streptomyces

Les expériences suivantes ont été mises en oeuvre avec divers champignons, ce sont :

Beauveria bassiana ATCC 7159
Aspergillus ochraceus ATCC 18500
Aspergillus foetidus ATCC 10254
Aspergillus allicaeus ATCC 10060
Aspergillus niger ATCC 9142
Stroptomyces Tü 96
Streptomyces incarnatus.

Les expériences préliminaires qui sont rapprotées ont été faites dans les conditions utilisées pour C. ech. (exemple 1).

Les pré-cultures ont été effectuées sur gélose inclinée de pomme de terre. Les cultures pnt été réalisées en milieu liquide "farine de soja" pendant 48 heures. Le DINIS a été ajouté (500 mg/l) au milieu de culture et incubé pendant 44 et 68 heures. Les taux de bioconversion ont été effectués par dosages par HPLC.

Les résultats obtenus (tableau 4) indiquent que :

1. Toutes les souches étudiées effectuent la conversion du DINIS en MONIS-5 et MONIS-2 en proportions variables et à des vitesses variables.

2. La souche Beauveria bassiana ATCC 7159 est remarquable ; elle conduit après 68 heures d'incubation à une conversion complète et quantitative du DINIS en MONIS-5 et MONIS-2 avec des rendements respectifs de 85 et 15 %.

TABLEAU 4

| Biotransformation du DINIS par différentes souches de champignons | | | | |
|---|---|---|---|---|
| Souche | Durée de l'incubation (heure) | Quantité de substance | | |
| | | MONIS-2 | MONIS-5 | DINIS |
| Beauveria bassiana | 44 | 46 | 252 | 110 |
| ATCC 7159 | 68 | 66 | 361 | 0 |
| Aspergillus foetidus | 44 | 18 | 31 | 430 |
| ATCC 10254 | 68 | 23 | 38 | 425 |
| Aspergillus alliaiens | 44 | 60 | 71 | 286 |
| ATCC 10060 | 68 | 61 | 97 | 212 |
| Aspergillus niger | 44 | 0 | 43 | 383 |
| ATCC 9142 | 68 | 10 | 58 | 377 |
| Aspergillus ochraceus | 44 | 0 | 27 | 396 |
| ATCC 18500 | 68 | 0 | 38 | 373 |
| Streptomyces Tü 96 | 44 | 0 | 21 | 497 |
| | 68 | 28 | 47 | 413 |
| Streptomyces | 44 | 20 | 70 | 400 |
| iscariatus | 68 | 53 | 100 | 360 |
| Le DINIS (500 mg/ml) a été additionné à la culture après 48 heures de pousse. L'évaluation du rendement tient compte de la perte du groupement $NO_2$ lors de la conversion DINIS (PM 236) en MONIS (PM 191). | | | | |

## EXEMPLE 4

1) Conditions de culture de la souche Beauveria bassiana (ATCC 7159)

1.1) Pré-cultures

Les pré-cultures sont effectuées à 24°C pendant 6 jours en tube d'agar sur 2 milieux au choix :
- gélose de pomme de terre (I.P.P.) ;
- milieu $M_2$ constitué de "Bacto Yeast extract" 4 g, "Bacto Malt extract" 10 g, "Bacto Dextrose" 4 g, agar 20 g pour 1 litre d'eau, pH 7, selon E.B. Shirling et D. Gottlieb (Intern. J. Syst. Bact. 16, 313-340, 1966).
Une sporulation plus abondante est observée sur milieu $M_2$.

1.2) Cultures

Les cultures de B. bassiana (B. b.) sont réalisées en milieu liquide "farine de soja" selon R.E. Betts, D.E. Walters, J.P. Rossaza (J. Med. Chem. 17, 597-602, 1974), constitué de farine de soja 5 g, glucose 20 g, "Yeast extract" 5 g, NaCl 5 g, $K_2HPO_4$ 5 g, sels minéraux $Fe^{+2}$ 0,1 mg/l, $Zn^{+2}$ 0,1 mg/l, $Mo^{+2}$ 0,01 mg/l, $Cu^{+2}$ 0,02 mg/l, $Mn^{+2}$ 0,01 mg/l, eau à pH 7.
50 ml de milieu sont ensemencés avec 0,5 à 1 ml d'une suspension de spores (500 000 à 300 000 spores/ml) préparée à partir d'un tube d'agar repris par 6 ml d'eau distillée. Les cultures effectuées dans des erlenmeyers de 250 ml sont agitées sur un agitateur rotatif à 180 tours/min. à 24°C.

2) Analyse des produits de biotransformation

Les concentrations relatives de DINIS, MONIS-5 et MONIS-2 ont été évaluées par HPLC.
L'analyse a été réalisée sur un appareil Waters équipé d'un détecteur U.V. (Pye Unicam) et d'un intégrateur Waters.
- colonne $\mu$Bondapack C18 (10 $\mu$m - 4 x 250 mm)

- solvant MeOH/$H_2O$ : 25/75
- débit 0,5 ml/min.
- détecteur U.V. $\lambda$ = 220 nm, sensibilité 0,08/5.

La séparation des 3 composés est satisfaisante :
- MONIS-5 : Rt = 10 min 20 sec
- MONIS-2 : Rt = 9 min
- DINIS : Rt = 25 min 20 sec.

La sensibilité très grande de la technique permet d'évaluer des quantités de substances de l'ordre de 60 ng (3 mg/l) jusqu'à 6 $\mu$g (300 mg/l) pour 20 $\mu$l injectés, donc des concentrations compatibles avec les conditions utilisées dans les essais de bioconversion. La réponse linéaire obtenue pour les trois composés entre la surface des pics et les quantités de substances injectées permet l'évaluation des concentrations des différents constituants présents dans le milieu d'incubation.

3) Extraction des constituants du milieu de culture

0,5 ml de milieu de culture après filtration (ou centrifugation) du mycelium est mis sur colonne en verre (diamètre 0,5 cm) remplie de 1 g de silice Kieselgel 60. On passe 10 ml d'éther éthylique. On évapore sous vide l'éther à sec, et dilue l'échantillon avec 2 ml du mélange MeOH/$H_2O$ (25/75). Cette solution est injectée directement dans un appareil HPLC. L'extraction est quantitative.

4) Etude des conditions de bioconversion du DINIS par Beauveria bassiana

D'une façon systématique, les études ont été faites sur des cultures de volume 50 ml, âgées de 48 heures, le DINIS a été ajouté en solution dans le méthanol à 50 mg/ml.

4.1) Incubation en milieu de culture : importance de la concentration du DINIS (0,5 et 1 g/l)

La bioconversion a été évaluée entre 17 et 45 heures d'incubation, les résultats obtenus sous une concentration de DINIS à 0,5 g/l (tableau 5) indiquent une bioconversion quasi complète dès 25 heures (quantité de DINIS résiduel 3 %), et totale à 38 heures, qui conduit à la formation du mélange MONIS-5 (85 %) et MONIS-2 (15 %), soit un rapport MONIS-5/MONIS-2 = 5,16/1, avec un rendement de 93 %.

TABLEAU 5

| Biotransformation du DINIS par Beauveria bassiana ATCC 7159 | | | |
|---|---|---|---|
| Temps (heure) | Quantité de substance | | |
| | MONIS-5 | MONIS-2 | DINIS |
| 17 | 108 | 33 | 361 |
| 18 | 190 | 48 | 304 |
| 25 | 307 | 62 | 15 |
| 38 | 315 | 61 | 0 |
| 45 | 290 | 59 | 0 |
| Incubation de B. bassiana en milieu de culture. Concentration du DINIS : 500 mg/ml. | | | |

Lorsque la concentration de DINIS est portée à 1 g/l pour la même quantité de Mycelium (tableau 6), 68 heures d'incubation sont nécessaires pour observer une consommation complète du DINIS, la formation du MONIS-2, MONIS-5 est effectuée avec un rendement global de 91 %. Cependant, cette bioconversion plus lente conduit au mélange MONIS-5/MONIS-2 = 3,9/1, donc moins favorable à l'obtention de l'isomère 5.

TABLEAU 6

| Biotransformation du DINIS par Beauveria bassiana ATCC 7159 | | | |
|---|---|---|---|
| Temps (heure) | Quantité de substance | | |
| | MONIS-5 | MONIS-2 | DINIS |
| 17 | 91 | 41 | 851 |
| 45 | 449 | 138 | 438 |
| 68 | 588 | 150 | 0 |
| Incubation de B. bassiana en milieu de culture. Concentration du DINIS : 1 g/l. | | | |

L'importance du rapport quantité de DINIS/quantité de mycelium pour la sélectivité de la biotransformation est illustrée par ces expériences.

4.2) Incubation dans l'eau

Une seconde série d'expériences a été effectuée dans l'eau. Ces conditions devaient permettre une extraction simplifiée des produits de bioconversion du milieu d'incubation dépourvu des constituants du milieu de culture.

Un mycelium obtenu à partir d'une culture de 48 heures a été mis en suspension dans de l'eau en présence d'une solution méthanolique de DINIS à la concentration finale de 1 g/l.

L'analyse du milieu de bioconversion (tableau 7) montre que dans ces conditions B. bassiana hydrolyse très lentement le DINIS. Après 30 heures d'incubation, 40 % de DINIS sont encore présents dans le milieu.

TABLEAU 7

| Biotransformation du DINIS par Beauveria bassiana ATCC 7159 | | | |
|---|---|---|---|
| Temps (heure) | Quantité de substance | | |
| | MONIS-5 | MONIS-2 | DINIS |
| 17 | 48 | 21 | 987 |
| 45 | 171 | 44 | 767 |
| 68 | 330 | 91 | 600 |
| 90 | 432 | 100 | 10 |
| Incubation de B. bassiana dans l'eau. DINIS : 1 g/l | | | |

**Revendications**

**1.** Procédé de bioconversion sélective de DINIS (dinitrate d'isosorbitol) en MONIS-5 (exo mononitrate d'isosorbitol), caractérisé en ce que le DINIS est ajouté à un milieu contenant au moins un microorganisme possédant une glutathion transférase ou un extrait acellulaire d'un tel microorganisme.

**2.** Procédé selon la revendication 1, caractérisé en ce que le DINIS est placé dans un milieu de culture contenant un microorganisme possédant une glutathion transférase.

**3.** Procédé selon l'une des revendications 1 et 2, caractérisé en ce que le microorganisme est choisi parmi les protozoaires, les fongis et les bactéries.

**4.** Procédé selon la revendication 3, caractérisé en ce que le microorganisme est choisi dans les genres : Tetrahymena, Cunninghamella, Aspergillus, Beauveria et Streptomyces.

5. Procédé selon l'une des revendications 1 à 4, caractérisé en ce que le microorganisme est choisi dans le genre Beauveria.

6. Procédé selon l'une des revendications 1 à 5, caractérisé en ce que la bioconversion est effectuée à une température de l'ordre de 20 à 30°C.

7. Procédé selon l'une des revendications 1 à 6, caractérisé en ce que le milieu est un milieu de culture minimum contenant de 5 à 20 g/l de glucose et une source d'azote organique contenant des acides aminés.

8. Procédé selon l'une des revendications 1 à 7, caractérisé en ce que le DINIS est ajouté à une concentration de 1 à 5 g/l de milieu.

9. Procédé selon l'une des revendications 1 à 8, caractérisé en ce que le pH du milieu est voisin de la neutralité.

## Claims

1. Process of selective bioconversion of DINIS (dinitrate of isosorbitol) into MONIS-5 exo mononitrate of isosorbitol, characterized in that the DINIS is added to a medium containing at least one microorganism posessing a glutathion transferase or a acellular extract of such a microorganism.

2. Process according to Claim 1, wherein the DINIS is placed in a culture medium containing a microorganism possessing a glutathion transferase.

3. Process according to Claim 1, wherein the microorganism is selected from among protozoa, fungi and bacteria.

4. Process according to Claim 3, wherein the microorganism is selected from the types: Tetrahymena, Cunninghamella, Aspergillus, Beauveria and Streptomyces.

5. Process according to Claim 1, wherein the microorganism is selected from the genus Beauveria.

6. Process according to Claim 1, wherein the bioconversion is carried out at a temperature of the order of 20 to 30°C.

7. Process according to Claim 1, wherein the medium is a minimum culture containing from 5 to 20 g/l of glucose and a source of organic nitrogen containing amino acids.

8. Process according to Claim 1, wherein the DINIS is added at a concentration of 1 to 5 g/l of medium.

9. Process according to Claim 1, wherein the pH of the medium is close to neutrality.

## Patentansprüche

1. Verfahren zur selektiven biologischen Umwandlung von DINIS (Isosorbitdinitrat) in MONIS-5 (Isosorbitexomononitrat), dadurch gekennzeichnet, daß das DINIS einem Medium zugesetzt wird, das mindestens einen Glutathiontransferase aufweisenden Mikroorganismus oder einen Zellextrakt eines solchen Mikroorganismus enthält.

2. Verfahren nach Anspruch 1, dadurch gekennzeichnet, daß das DINIS in ein Kulturmedium eingebracht wird, das einen eine Glutathiontransferase aufweisenden Mikroorganismus enthält.

3. Verfahren nach einem der Ansprüche 1 und 2, dadurch gekennzeichnet, daß der Mikroorganismus ausgewählt wird unter den Protozoen, den Pilzen und den Bakterien.

4. Verfahren nach Anspruch 3, dadurch gekennzeichnet, daß der Mikroorganismus ausgewählt wird unter den Genus Tetrahymena, Cunnighamella, Aspergillus, Beauveria und Streptomyces.

**5.** Verfahren nach einem der Ansprüche 1 bis 4, dadurch gekennzeichnet, daß der Mikroorganismus aus dem Genus Beauveria ausgewählt wird.

**6.** Verfahren nach einem der Ansprüche 1 bis 5, dadurch gekennzeichnet, daß die biologische Umwandlung bei einer Temperatur in der Größenordnung von 20 bis 30°C durchgeführt wird.

**7.** Verfahren nach einem der Ansprüche 1 bis 6, dadurch gekennzeichnet, daß es sich bei dem Medium um ein Kulturmedium handelt, das 5 bis 20 g/l Glucose und eine Aminosäuren enthaltende organische Stickstoffquelle enthält.

**8.** Verfahren nach einem der Ansprüche 1 bis 7, dadurch gekennzeichnet, daß das DINIS in einer Konzentration von 1 bis 5 g/l Medium zugesetzt wird.

**9.** Verfahren nach einem der Ansprüche 1 bis 8, dadurch gekennzeichnet, daß der pH-Wert des Mediums nahe bei dem Neutralpunkt liegt.